Europäisches Patentamt

European Patent Office

Office européen des brevets

(11) Publication number: **0 330 359**
**A2**

# (12) EUROPEAN PATENT APPLICATION

(21) Application number: 89301364.9

(22) Date of filing: 14.02.89

(51) Int. Cl.⁴: **C07K 7/08 , A61K 39/21 , A61K 39/42 , C12P 21/00 , G01N 33/569 , C12N 15/00 , //C07K1/04**

Claims for the following Contracting State: ES.

(30) Priority: **25.02.88 US 160378**

(43) Date of publication of application:
**30.08.89 Bulletin 89/35**

(84) Designated Contracting States:
**CH DE ES FR GB IT LI**

(71) Applicant: **BIO-RAD LABORATORIES, INC.**
**1414 Harbor Way South**
**Richmond California 94804(US)**

(72) Inventor: **Walker, Roger P.**
**861 Oxford Way**
**Benecia California 94510(US)**
Inventor: **Parekh, Bharat S.**
**100 Ellinwood Drive**
**No.131D Pleasant Hill California 94523(US)**

(74) Representative: **Paget, Hugh Charles Edward**
**et al**
**MEWBURN ELLIS & CO. 2/3 Cursitor Street**
**London EC4A 1BQ(GB)**

(54) Composition useful in the diagnosis and treating of HIV-1 infection.

(57) Methods and compositions for HIV-1 diagnosis, treatment, and vaccination include one or more peptides which correspond to antigenic determinants encoded by conserved regions of the HIV-1 genome. The peptides will be immunologically cross-reactive with such antigenic determinant sites and, preferably, will be capable of eliciting a serum-neutralizing response when administered to an individual who may or may not be infected with HIV-1. Antibodies to such peptides will also find use in the present invention.

EP 0 330 359 A2

## COMPOSITIONS USEFUL IN THE DIAGNOSIS AND TREATING OF HIV-1 INFECTION

The present invention relates generally to the diagnosis and treatment of viral infections, and more particularly to the preparation of compositions useful for the diagnosis of and immunization against human immunodeficiency virus (HIV-1) infection.

Human immunodeficiency virus (HIV-1), previously designated HTLV-III and lymphoadenopathy-associated virus (LAV), has now been identified as the etiological agent responsible for acquired immunodeficiency syndrome (AIDS). As the spread of AIDS threatens to become the most deadly infectious disease of the twentieth century, methods for diagnosing and immunizing against HIV-1 infection become increasingly important.

A particular problem in both HIV-1 detection and immunization has been the considerable genetic variability of the virus. HIV-1 comprises numerous variants, differing by as few as 80 nucleotides and as many as 1000 or more nucleotides. As the HIV-1 genome comprises only 9500 base pairs, it would be expected that there will be considerable variations in the antigenic characteristics of the various strains. Such variations in the antigenic character of the various strains, in turn, render the task of preparing effective diagnostic and therapeutic compositions much more difficult.

One hopeful approach for the preparation of HIV-1 diagnostics and vaccines has been to identify conserved or non-variable regions among the genomes of the various strains. Such conserved regions which encode immunologically recognizable antigens on the viral core and envelope would ideally serve as the basis for preparing immunological reagents which are cross-reactive with a wide range or preferably all strains of HIV-1.

Unfortunately, while numerous peptides corresponding to conserved regions within the HIV-1 genome have been identified, none have yet provided entirely reliable diagnostic reagents. Moreover, none of the conserved peptides have been proven to be at all effective in preparing vaccines or other therapeutic compositions.

For these reasons, it would be desirable to identify additional conserved regions within the HIV-1 genome which encode viral antigens which are immunologically reactive on a large number, preferably all, of the HIV-1 strains. Alternatively, it would be desirable to identify groups of conserved or non-conserved peptides which, when the individual peptides of the group are employed in combination, can be used as reagents in detecting the presence of HIV-1 antibodies to HIV-1 in patient samples. It would be particularly desirable if such conserved peptides or groups of peptides could be effectively employed in vaccines, typically referred to as subunit vaccines, where they could be used to elicit serum-neutralizing antibodies capable of reacting with a large number of HIV-1 strains when administered to individuals as a vaccine.

Preparation of HIV-1 peptides is described in several references. A 21 amino acid peptide corresponding to a sequence encoded by gp41 is described in European Patent Application No. 86/303224.9, Wang et al. (1987) ACPR 21:34, and Proc. Natl. Acad. Sci. USA 83:6159-6163. Kennedy et al. (1986) Science 231:1556-1559 describe an 18 amino acid peptide corresponding to a sequence encoded by gp160, the precursor to gp20 and gp41. Rosen et al. (1987) in: "Clinical Laboratory Molecular Analysis:Future Directions" pp. 229-236 and Smith et al. (1987) J. Clin. Microbiol. 25:1498-1504 describe the preparation of various peptides encoded by various regions on the gag and env genes. Wong-Staal et al. (1986) in U.S. Patent Application Serial No. 779,431 (available from NTIS) describe 14 and 17 amino acid peptides which are reactive with HIV-1. U.S. Patent No. 4,629,783 describes a number of peptides corresponding to sequences on gp110 and gp25 which are reactive with HIV-1. Shoeman et al. (1987) Anal. Biochem. 161:370-379 describe several peptides encoded by portions of the gag and env genes which are reactive with HIV-1.

Peptides corresponding substantially to epitopic or antigenic determinant sites encoded by the HIV-1 genome are useful in formulating diagnostic, therapeutic, and vaccine compositions. The peptides may be highly conserved within the various strains of HIV-1, or may be less conserved peptides where it is desired to utilize cross-reactivity with a limited number (i.e., fewer than all) of HIV-1 strains. The less conserved peptides may also find use in combination with other more or less conserved peptides where the combination of peptides is cross-reactive with most or all HIV-1 strains. Preferably, the peptides or groups of peptides will be able to elicit a serum neutralizing immune response when administered to a normal or HIV-1-infected individual. Additionally, antibodies prepared against the peptides or groups of peptides will be useful in formulating diagnostic and therapeutic compositions and may be used to affinity purify monospecific antibodies from polyclonal sera.

In a preferred aspect of the present invention, the peptides are synthetically-produced linear fragments having a length in the range from about 6 to 50 amino acids, usually being in the range from about 8 to 25 amino acids. Use of such synthetic fragments is advantageous in that large quantities of the peptides may be produced without purification from an HIV-1 source. Moreover, the synthetic peptides are free from bacterial and yeast components which are normally present in recombinantly-produced peptides. Surprisingly, a plurality of linear fragments have been identified which are cross-reactive with high percentages of HIV-1 strains derived from diverse geographical locations. These linear fragments may be employed in combination to identify HIV-1-infected sera employing conventional immunoassays, particularly ELISA'S and dot blots. Usually, at least two immunologically distinct peptides will be employed in the assay procedure, more usually at least four, and frequently eight or more. Such assays may be employed as well for pathogens other than HIV-1, including both viral and bacterial pathogens.

In a second preferred aspect of the present invention, conserved peptides encoded by the regulatory regions of the HIV-1 genome have been prepared and used to screen for HIV-1-infected sera. Detection of antibodies to the regulatory proteins in sera appears to be highly diagnostic of HIV-1 infection and may show a correlation with disease state. Particular peptides encoded by the tat, 3'orf, Rorf, Uorf, trs/art and sor regions of the HIV-1 genome have been identified.

## DESCRIPTION OF THE PREFERRED EMBODIMENTS

According to the present invention, novel compositions and methods are provided for detecting, inhibiting, and neutralizing HIV-1 infection. The compositions include peptides having an amino acid sequence which is substantially the same as that encoded by various conserved and non-conserved regions of the HIV-1 genome. The compositions also comprise groups of peptides corresponding to immunologically distinct regions on the HIV-1 regulatory proteins. The individual peptides are selected so that the groups are immunologically cross-reactive with most and preferably all strains of HIV-1. The peptides and groups of peptides will correspond to immunologically-reactive sites on the HIV-1 particle, and preferably will mimic a neutralizing region on the virus. The compositions of the present invention also include antibodies raised against the peptides.

HIV-1 is classed as a retrovirus and includes a ribonucleoprotein core surrounded by a lipid-containing envelope. The HIV-1 genome is more complex than most other known retroviruses, and each end of the provirus includes long terminal repeats which regulate transcription. At least seven genes are encoded by the genome, including the gag, pol, and env genes which encode the core proteins, reverse transcriptase, and envelope proteins respectively. The core proteins include g18, while the envelope proteins include gp41 and gp120, which are derived from precursor gp160. The genes encoding the regulatory proteins include tat, orf, trs/art, and sor. The tat gene appears to regulate the transcription and possibly the translation of mRNA. The trs gene appears to affect the distribution of the various forms of mRNA, and promote structural protein synthesis. The roles of the orf and sor genes are not known.

The term "neutralizing region" refers to those portions of the HIV-1 genome which encode epitopes reactive with antibodies which are capable of neutralizing HIV-1 infection in a human host. Suitable assays for neutralization are well known and may be based on reduction of HIV-1 infection in T-cell lines, reduction of plaque forming units (pfu) of HIV-1 pseudotypes bearing the envelope or core glycoproteins of HIV-1, syncytial inhibition tests, and virion-receptor binding tests. Alternatively, the neutralizing activity can be compared to antibody reactivity in immunochemical tests, such as immunofluorescence, immunoblot, and radioimmunoprecipitation assays.

The peptides of interest correspond to certain amino acid sequences encoded by the env, gag, pol, tat, orf, trs/art, and sor regions of the HIV-1 genome. More specifically, the peptides are fragments of gp120 and gp41 encoded by the env gene, p18 and p24 encoded by the gag gene, p32 encoded by the pol gene, and the regulatory proteins encoded by the tat, orf, trs/art, and sor genes. The sequences and locations. within the genes for the particular peptides are set forth in Table 1.

3

## TABLE 1

| Peptide Designation | Gene | Amino Protein | Amino Acid Acid Nos. | Sequence* |
|---|---|---|---|---|
| 3 | gag | p18 | 1-15 | MGARASVLSGGELDP |
| 4 | gag | p18 | 11-25 | GELORWEKIRLRPGG |
| 5 | gag | p18 | 21-35 | LRPGGKKKYKLKHIV |
| 7 | gag | p18 | 41-55 | LERFAVNPGLLETSE |
| 9 | gag | p18 | 61-75 | LGQLQPSLQTGSEEL |
| 10 | gag | p18 | 71-85 | GSEELRSLTNTVATL |
| 11 | gag | p18 | 81-95 | TVATLYCVHQRIEIK |
| 12 | gag | p18 | 91-105 | RIEIKDTKEALDKIE |
| 23 | gag | p24 | 221-235 | GPIAPGQMREPRGSD |
| 24 | gag | p24 | 231-245 | PRGSDIAGTTSTLQE |
| 25 | gag | p24 | 241-255 | STLQEQIGWMTNNPP |
| 26 | gag | p24 | 251-265 | TNNPPIPVGEITKRW |
| 28 | gag | p24 | 261-275 | IYKRWIILGLNKIVR |
| 36 | gag | p24 | 351-365 | QGVGGPGHKARVLAE |
| 37 | gag | p24 | 361-375 | RVLAEAMSQVTNTAT |

4

| Peptide Designation | Gene | Amino Protein | Amino Acid Acid Nos. | Sequence* |
|---|---|---|---|---|
| 180 | gag | p15 | 398-412 | EGHTARNCRAPRKKG |
| 181 | gag | p15 | 408-422 | PRKKGCWKCGKEGHQ |
| 182 | gag | p15 | 418-432 | KEGHQMKDCTERQAN |
| 184 | gag | p15 | 438-452 | WPSYKGRPGNPLQSR |
| 189 | gag | p15 | 488-502 | QEPIDKELYPLTSLR |
| 40 | env | gp120 | 11-25 | WRWGWRWGTMLLGML |
| 42 | env | gp120 | 31-45 | TEKLWVTVYYGVPVW |
| 43 | env | gp120 | 41-55 | GVPVWKGATTTLFCA |
| 50 | env | gp120 | 110-125 | LWDQSLKPCVKLTPL |
| 51 | env | gp120 | 121-135 | KLTPLCVSLKCTDLK |
| 52 | env | gp120 | 131-145 | CTDLKNDTNTNSSSG |
| 55 | env | gp120 | 161-175 | ISTSIRGKVQKEYAF |
| 61 | env | gp120 | 231-245 | KTFNGTGPCTNVSTV |
| 62 | env | gp120 | 241-255 | NVSTVQCTHGIRPVV |
| 63 | env | gp120 | 251-265 | IRPVVSTQLLLNGSL |
| 68 | env | gp120 | 301-315 | NNTRKSIRIQRGPGR |
| 82 | env | gp120 | 441-455 | GQIRSCCNITGLLLT |
| 84 | env | gp120 | 461-475 | SNNESEIFRPGGGDM |
| 87 | env | gp120 | 491-505 | IEPLGVAPTKAKRRV |
| 88 | env | gp120 | 501-515 | AKRRVVQREKRAVGI |
| 89 | env | gp41 | 511-525 | RAVGIGALFLGFLGA |
| 90 | env | gp41 | 521-535 | GFLGAAGSTMGAASM |
| 91 | env | gp41 | 531-545 | GAASMTLTVQARQLL |
| 92 | env | gp41 | 541-555 | ARQLLSGIVQQQNNL |
| 93 | env | gp41 | 551-565 | QQNNLLRAIEAQQHL |
| 94 | env | gp41 | 561-575 | AQQHLLQLTVWGIKQ |
| 99 | env | gp41 | 591-605 | QLLGIWGCSGKCICT |
| 100 | env | gp41 | 601-615 | KLICTTAVPWNASWS |
| 101 | env | gp41 | 611-625 | NASWSNKSLEQIWNN |
| 102 | env | gp41 | 621-635 | QIWNNMTWMEWDREI |
| 103 | env | gp41 | 631-645 | WDREINNYTSLIHSL |

| Peptide Designation | Gene | Amino Protein | Amino Acid Acid Nos. | Sequence* |
|---|---|---|---|---|
| 105 | env | gp41 | 651-665 | NQQEKNEQELLELDK |
| 106 | env | gp41 | 661-675 | LELDKWASLWNWFNI |
| 107 | env | gp41 | 671-685 | NWFNITNWLWYIKLF |
| 108 | env | gp41 | 681-695 | YIKLFIMIVGGLVGL |
| 112 | env | gp41 | 721-735 | LPIPRGPDRPEGIEE |
| 114 | env | gp41 | 741-755 | DRDRSIRLVNGSLAL |
| 119 | env | gp41 | 791-805 | EALKYWWNLLQYWSQ |
| 120 | env | gp41 | 801-815 | QYWSQELKNSAVSLL |
| 122 | env | gp41 | 821-835 | AVAEGTDRVIEVVQG |
| 130 | pol | p32 | 625-639 | VTNKGRQKVVPLTNT |
| 131 | pol | p32 | 635-649 | PLTNTTNQKTELQAI |
| 132 | pol | p32 | 645-659 | ELQAIYLALQDSGLE |
| 139 | pol | p32 | 715-729 | VDKLVSAGIRKILFL |
| 143 | pol | p32 | 755-769 | LPPVVAKEIVASCDK |
| 144 | pol | p32 | 765-779 | ASCDKCQLKGEAMHG |
| 147 | pol | p32 | 795-809 | LEGKVILVAVHVASG |
| 155 | pol | p32 | 875-889 | QGVVESMNKELKKII |
| 156 | pol | p32 | 885-899 | LKKIIGQVRDQAEHL |
| 167 | pol | p32 | 995-1009 | IRDVGKQMAGDDCVA |
| 168 | pol | p32 | 1000-1015 | QMAGDDCVASRQDED |
| 169 | tat | p__ | 1-15 | MEPVDPRLEPWKHPG |
| 383 | u-orf | p27 | 1-15 | MQPIQIAIVALVVAI |
| 384 | u-orf | p27 | 11-25 | LVVAIIIAIVVWSIV |
| 388 | u-orf | p27 | 31-45 | KILRQRKIDRLIDRL |
| 389 | u-orf | p27 | 41-55 | LIDRLIERAEDSGNE |
| 393 | r-orf | p__ | 1-15 | MEQAPEDQGPQREPH |
| 394 | r-orf | p__ | 11-25 | QREPHNEWTLELLEE |
| 397 | r-orf | p__ | 41-55 | GLGQHIYETYGDTWA |

6

| Peptide Designation | Gene | Amino Protein | Amino Acid Acid Nos. | Sequence* |
|---|---|---|---|---|
| 403 | trs/art | p__ | 21-35 | FLYQSNPPPNPEGTR |
| 406 | trs/art | p__ | 51-65 | QIHSISERILGTYLG |
| 409 | trs/art | p__ | 81-95 | LTLDCNEDCGTSGTQ |
| 420 | sor | p23 | 71-85 | GLHTGERDWHLGQGV |
| 421 | sor | p23 | 81-95 | LGQGVSIEWRKKRYS |
| 422 | sor | p23 | 91-105 | KKRYSTQVDPELADQ |
| 423 | sor | p23 | 101-115 | ELADQLIHLYYFDCF |
| 427 | sor | p23 | 141-155 | KVGSLQYLALAALIT |
| 428 | sor | p23 | 151-165 | AALITPKKIKPPLPS |
| 429 | sor | p23 | 161-175 | PPLPSVTKLTEDRWN |

\*     Nucleotide and amino acid sequence numbering is based on the published sequence for the HBX2 and BH10 strains (Human Retrovirus and AIDS, Myers et al., eds., Los Alamos National Laboratory, Los Alamos, New Mexico 87545 (1987)).

The amino acid sequences of the peptides of the present invention need not correspond precisely to the sequences set forth in the above table. Rather, it is essential only that the peptides define at least one epitope or antigenic determinant which is immunologically cross-reactive with the enumerated sequences. Thus, peptides of the present invention may include only a portion of the listed amino acid sequences, but must include at least 6 contiguous amino acids, usually including at least 9 amino acids, and typically including at least 12 or more amino acids of any one of the sequences.

The peptides of the present invention may also include additional amino acid sequences not corresponding to those enumerated in the above Table. Such additional sequences may be present at either the C-terminus, N-terminus, or both and can either be synthesized at the same time as the listed sequences, or may be conjugated or attached to the sequences by well known techniques. Usually, such additional sequences may be useful in separation, conjugation, or other manipulation of the peptides which facilitate their use in some known manner. In particular, the peptides may include additional amino acids or be otherwise modified at the C-terminus or N-terminus to provide for binding or conjugation of the peptide to a solid phase or another protein. For example, a gly-gly-cys sequence may be added to either terminus to facilitate coupling to a carrier. Hydrophobic residues or lipid-containing moieties may be added to enhance liposome or membrane binding. While there is no theoretical upper limit to the size of the peptides of the present invention, there will seldom be reason to prepare peptides having a length greater than 100 amino acids, and the peptides will usually be shorter than 75 amino acids.

The peptides of the present invention may also embody substitutions of particular amino acids, although there will usually be no substitutions in shorter peptides of only six amino acids. Normally, there will be no more than one substitution in sequences from 6 to about 10 amino acids, and no more than two substitutions in sequences having a length from about 10 to 20 amino acids. Substitutions, of course, must not substantially alter the immunological characteristic of the peptide in an adverse manner and may result in enhanced binding or antigenic properties. The cross-reactivity with the particular epitope or antigenic determinant site must not be substantially reduced.

Depending on its length and location, the peptide of the present invention may be substantially free from folding, may display folding which is not characteristic of the natural gene product, or may display a natural or substantially natural folding. Usually, the shorter fragments of 25 amino acids and below will display minimal or non-natural folding, while the longer fragments are more likely to display folding which is similar to the natural product.

The peptides of the present invention may also be incorporated in compositions for the prophylactic and/or therapeutic treatment of HIV-1 infection. In therapeutic applications, the compositions are administered to a patient already infected with HIV-1, in an amount sufficient to cure or at least partially inhibit the infection and its complications. An amount adequate to accomplish this is defined as a therapeutically effective dose. Precise determination of such a dose will depend on the severity of the infection and the general state of the patient's own immune system, but will generally range from about 1 to 200 milligrams of peptide per kilogram of body weight, typically being in the range from about 5 to 25 milligrams per kilogram of body weight.

In prophylactic applications, the peptides of the present invention are administered to an individual who is not infected with HIV-1, but who will usually be at risk of being exposed to the virus. In such prophylactic applications, the peptides will be formulated in a vaccine composition intended to elicit a protective immune response. The vaccine compositions will include a prophylactically effective dose, which will again depend on the patient's state of health and general level of immunity. Usually, the dosage will be somewhat less than for therapeutic applications, typically being in the range from about 0.1 milligrams to 25 milligrams per kilogram of body weight, usually being in the range from about 0.5 milligrams to 2.5 milligrams per kilogram of body weight.

Single of multiple administrations of the peptide compositions may be carried out with the exact dose level and pattern of administration being selected by the treating physician.

The peptides will be incorporated in a physiologically-acceptable carrier, and will be injected intravenously, subcutaneously, intramuscularly, intraperitoneally, or the like. Suitable carriers include phosphate buffered saline, saline, water, potassium chloride, sodium lactate, and the like. The concentration of the peptide in the carrier will vary depending on the ultimate use, activity, and mode of administration.

The peptides of the present invention may be natural, i.e., fragments of protein isolated from HIV-1, but will more usually be synthetic. The natural proteins may be isolated from HIV-1 by conventional techniques such as high performance liquid chromatography (HPLC) and affinity chromatography, but care should be taken that no intact HIV-1 or infectious materials are left in the isolated compositions. Conveniently, polyclonal or monoclonal antibodies obtained according to the present invention (as described below) may be used to prepare a suitable affinity column by well known techniques. Such techniques are taught, for example, in Hudson and Hay, Practical Immunology, Blackwell Scientific Publications, Oxford, United Kingdom, 1980, Chapter 8. The peptides may then be obtained by chemical or enzymatic cleavage of the intact protein.

Because of the difficulty and danger in isolating the peptides of the present invention from HIV-1, however, it is preferred to produce synthetic fragments based on the HIV-1 amino acid sequences as set forth above. Synthetic polypeptides which are immunologically cross-reactive with the natural HIV-1 proteins may be produced by either of two general approaches. First, polypeptides having fewer than about 100 amino acids, usually fewer than about 50 amino acids, and more usually 25 or fewer amino acids, may be synthesized by the well-known Merrifield solid-phase chemical synthesis method where amino acids are sequentially added to a growing chain (Merrifield (1963) J. Am. Chem. Soc. 85:2149-2156). Specific synthesis techniques are described below.

Linear peptides may be chemically synthesized by manual means or automatically in commercially-available synthesis equipment. Systems for manually synthesizing peptides on polyethylene pegs are available from Cambridge Research Biochemicals. An exemplary manual technique for preparing peptides is described in detail in the Experimental section hereinafter. Automatic peptide synthesis equipment is available from suppliers including Applied Biosystems, Inc., Foster City, California, Beckman Instruments, Inc., Waldwick, New Jersey, and Biosearch, Inc., San Rafael, California. Using such automatic synthesizers according to manufacturer's instructions, peptides may be produced in gram quantities for use in the present invention.

The use of relatively short linear peptide fragments has a number of advantages in performing the methods of the present invention. The peptides may be produced in quantity and free from contaminating substances which are found in recombinantly produced peptides. Moreover, the risks associated with obtaining peptides from HIV-1 itself are entirely avoided. Short, linear peptides are particularly effective when employed in groups of at least two, frequently four, and often eight or more. By properly selecting the peptides from different conserved and non-conserved regions of the HIV-1 genome, assays can be devised which are capable of detecting infection with most and preferably all HIV-1 strains. Specific assay protocols utilizing groups of linear peptide fragments are described in more detail hereinafter.

The second general approach for synthesizing the peptides of the present invention involves the expression in cultured cells of recombinant DNA molecules encoding a desired portion of the HIV-1 env, gag or pol gene. The HIV-1 gene may itself be natural or synthetic. Conveniently, polynucleotides may be synthesized by well-known techniques. For example, short single-stranded DNA fragments may be prepared by the phosphoramidite method described by Beaucage and Carruthers (1981) Tett. Letters 22:1859-1862. A double-stranded fragment may then be obtained either by synthesizing the complementary strand and annealing the strands together under appropriate conditions or by adding the complementary strand using DNA polymerase with an appropriate primer sequence.

The natural or synthetic DNA fragments coding for a desired HIV-1 fragment will be incorporated in DNA constructs capable of introduction to and expression in an in vitro cell culture. Usually, the DNA constructs will be suitable for replication in a unicellular host, such as yeast or bacteria, but may also be intended for introduction and integration within the genome of cultured mammalian or other eukaryotic cell lines. DNA constructs prepared for introduction into bacteria or yeast will include a replication system recognized by the host, the HIV-1 DNA fragment encoding the desired polypeptide product, transcriptional and translational initiation regulatory sequences joined to the 5′-end of the HIV-1 DNA sequence, and transcriptional and translational termination regulatory sequences joined to the 3′-end of the HIV-1 sequence. The transcriptional regulatory sequences will include a heterologous promoter which is recognized by the host. Conveniently, available expression vectors which include the replication system and transcriptional and translational regulatory sequences together with an insertion site for the HIV-1 DNA sequence may be employed.

To be useful in the detection and immunization methods of the present invention, the peptides are obtained in substantially pure form, that is, typically about 50% w/w or more purity, substantially free of interfering proteins and contaminants. Preferably, the HIV-1 peptides are isolated or synthesized in a purity of at least about 80% w/w and, more preferably, in at least about 95% w/w purity. Using conventional purification techniques, homogeneous peptides of at least 99% w/w can be obtained. For example, the peptides may be purified by use of reverse-phase high performance liquid chromatography. Usually, however, it is not essential to obtain highly pure peptide, particularly when using the peptides as a binding reagent in diagnostic assays.

Antibodies to the HIV-1 peptides may be obtained by injecting the purified peptide into a wide variety of vertebrates in accordance with conventional techniques. Suitable vertebrates include mice, rats, sheep, and goats, and in particular mice. Usually, the animals are bled periodically with successive bleeds having improved titer and specificity. The antigens may be injected intramuscularly, intraperitoneally, subcutaneously, or the like. Usually, a vehicle is employed, such as a complete or incomplete Freund's adjuvant. If desired, monoclonal antibodies can be prepared. Alternatively, the peptides may be used to isolate monospecific antibodies from a natural source, i.e., from the sera of an individual infected with HIV-1.

To obtain monoclonal antibodies, spleen cells from the immunized vertebrate are immortalized. The manner of immortalization is not critical. Presently, the most common method is fusion with a myeloma fusion partner. Other techniques include EBV transformation, transformation with bare DNA, e.g., oncogenes, retroviruses, etc., or any other method which provides for stable maintenance of the cell line and production of monoclonal antibodies. Human monoclonal antibodies may be obtained by fusion of the spleen cells with an appropriate human fusion partner. A detailed technique for producing mouse x mouse monoclonal antibodies is taught by Oi and Herzenberg, in "Selected Methods in Cellular Immunology," Mishell and Shiigi (eds.), W. H. Freeman and Co., San Francisco (1980), pp. 351-372. The antibodies of the present invention may be of any immunoglobulin class, i.e, IgG, including IgG1, IgG2A, and IgG2B, IgA, IgD, IgE, and IgM, usually being IgG or IgM.

Once antibodies having suitable specificity have been prepared, a wide variety of immunological assay methods are available for detecting the HIV-1 in a biological sample. Numerous competitive and non-competitive protein binding assays have been described in the scientific and patent literature, and a large number of such assays are commercially available.

In performing immunoassays according to the present invention, it will usually be necessary to pretreat the biological sample in some manner. Sample preparation will vary depending on the source of the biological sample. Serum samples will typically be prepared by clotting whole blood and isolating the supernatants in accordance with well known methods. Other biological fluids, such as semen, sputum, and urine, may also be prepared by conventional techniques. Solid tumors and other tissue samples will usually be prepared by lysing the cells and solubilizing the cellular components of interest.

The peptides of the present invention are particularly useful as reagents in solid phase binding assays where antibodies in a patient sample are immobilized by peptides bound to a solid phase. While patient samples may be screened against a single peptide, it will normally be preferred to utilize a group or panel of peptides where the individual peptides in the group are selected to maximize the probability of binding

9

HIV-1 antibodies. Because of the heterogeneity of the HIV-1 strains and variations in an individual's immune response (i.e., certain individual patients may be incapable of producing a detectable level of antibodies to particular epitopes on the viral particle), detection based on antibody reactivity with a single peptide will generally be inadequate.

The individual peptides in such groups will also be selected to avoid cross-reactivity with viruses other than HIV-1, such as HIV-2. The avoidance of such cross-reactivity is critical in avoiding false positive test results.

The individual peptides in such groups may be employed simultaneously or discretely. Normally, the peptides will be mixed and attached to a single solid phase and the assays then run in a conventional manner (exemplary protocols are then set forth below). Binding of antibody in the patient sample to one or more of the peptides will result in a positive test, while failure of all peptides to bind antibody is a highly reliable indication that the patient is free from HIV-1 antibodies. In some cases, however, it may be desirable to separately test for the reactivity of each individual peptide, or a subgrouping of peptides. The reactivity profiles obtained by such individual screening will provide more detailed information on the patient's immune response which may be of substantial value.

The present invention also provides for the detection of HIV-1 regulatory proteins in patient samples, particularly serum samples. The regulatory proteins may be detected simultaneously with or separately from the structural peptides. Because of the relatively low concentration of the regulatory proteins in the patient serum, it will usually be desirable to utilize high regulatory peptide concentrations on the solid phase to assure detection of antibodies in the patient sample.

The peptides and antibodies of the present invention are useful in virtually any type of immunological detection, including homogeneous and non-homogeneous (solid phase) immunoassays, competitive and non-competitive assays, and immunohistochemical staining techniques. Preferred are dot blot and enzyme-linked immunosorbent assays (ELISA), and specific protocols for such assays are set forth hereinbelow.

The following examples are offered by way of illustration, not by way of limitation.


## EXPERIMENTAL


Two hundred thirty five peptides 15 amino acids in length were synthesized according to the simultaneous multiple peptide synthesis method of Geysen et al. (1985) Proc. Natl. Acad. Sci. USA 81:3998 and 82:178, on polyethylene rods functionalized with a six-carbon aliphatic diamine spacer coupled to FMOC-$\beta$-alanine. The rods were obtained from Cambridge Research Biochemicals, Cambridge, United Kingdom. FMOC-protected amino acids active esters were added to the spacer, where the active esters were pentafluorophenyl esters except for serine and threonine which were 1-oxo-2-dihydroxyldihydrobenzotriazine esters. Following the synthesis, the peptide side-chain protecting groups were removed, leaving the peptides still attached to the supports. The sequences of the peptides were based on the predicted sequence of HIV-1 strains HBX2 and BH10 and selected to cover the entire amino acid sequence of all HIV-1 gene products. Consecutive peptides overlapped by five amino acids, and each peptide was tested for reactivity with a panel of HIV-1 positive and negative sera. The test was performed according to the ELISA protocol set forth hereinafter. Of the 235 peptides tested, 84 were found to react with at least 60% of the HIV-1 positive samples and with none of the HIV-1 negative samples. These 84 peptides are listed in Table 1 hereinabove.

Selected ones of the 84 peptides were then tested as follows. PEG pins containing HIV-1 synthetic peptide 15-mers were preincubated with Blotto solution for 30 minutes on a shaker at room temperature (RT). Pins were then washed twice with PBS-Tween®. These pins were immersed into the wells of a 96-well microtiter plate containing in each well 175 $\mu$l of HIV-1-positive or negative sera diluted to 1/100. After 1 hour of incubation at room temperature on a shaker, pins were initially washed with PBS-Tween® supplemented with 0.3 M NaCl in order to reduce the non-specific binding of human Ig onto pins. This was followed by two washes with PBS-Tween®. Then, the pins were again immersed into the wells of a 96-well microtiter plate containing 175 $\mu$l of anti-human Ig-alkaline phosphatase (Bio-Rad) for 30 minutes at RT on a shaker. Again, the pins were washed with PBS-Tween® supplemented with 0.3 M NaCl once, followed by two washes with PBS-Tween®. The substrate (p-nitro phenyl phosphate) dissolved in diethanolamine buffer (Bio-Rad) was added into the wells of 96-well microtiter plate (150 $\mu$l) and pins were immersed into these wells for 30 minutes at 37°C. The absorbance at 405 nm was recorded using a Titertek Plate Reader connected to the Microvax computer. Positivity was determined from the absorbance values above the background read from the bar graphs. Individual HIV-1 positive and negative sera were used except for HLR P I, II and III, which each was a pool of ten individual positive sera obtained from Hoffman-La Roche.

Since the peptide-containing polyethylene rods were re-used in the screening of several antisera, they were cleaned of any adhering antibodies or proteins by heating at 60°C disruption buffer for 30 minutes. This buffer consisted of 1% sodium dodecyl sulfate and 0.1% 2-mercaptoethanol in sodium phosphate buffer, pH 7.2. Following a final wash in boiling methanol, the rods were ready for re-use.

In order to test each more thoroughly, the 84 peptides of Table 1 were synthesized in greater quantity by conventional automated and manual solid-phase techniques. The peptides were evaluated for their utility as binding reagents in both ELISA and dot blot assays.

The peptides were used in dot blot membrane assays for the detection of HIV-1 specific antibodies in human sera as follows. The peptides were dissolved in phosphate-buffered saline (PBS) containing 0.1% sodium azide at a concentration of 2 mg/ml. Approximately 1 microliter of this solution was spotted directly onto a nitrocellulose membrane (Gelman), and the membranes dried at 45° for 2 hours. The membrane was washed twice for 20 minutes each with PBS containing 0.3% Tween®-20 and then distilled water.

A 1% dilution of individual sera in wash diluent (0.5% non-fat dry milk powder in PBS containing 0.3% Tween®-20) was incubated over the peptide-spotted membranes for 60 minutes at room temperature. Unbound antibodies were removed by two 10-minute washes with wash diluent. Subsequently, goat anti-human IgG alkaline phosphatase conjugate in wash diluent was allowed to react for 30 minutes. Following two 10-minute washes with wash diluent, the membranes were incubated with 5-bromo-4-chloro-3-indolyl phosphate (BCIP) and nitroblue tetrazolium (NBT) for 10 minutes. Positive sera were detected by the development of a purple color at the position of the peptide on the membrane. Negative sera showed no color change.

ELISA assays were performed as follows. Microtiter plates (96 well, Immulon-II) were coated with one or more peptides (250 ng - 1000 ng in 100 µl of PBS per well) by incubation at room temperature for 16-20 hours. The wells were then washed three times with PBS containing 0.3% Tween®-20. Serum samples were diluted 1:100 µl with wash diluent and 100 µl of sample was added to each well. After incubation at room temperature for 1 hour, the wells were washed six times with PBS containing 0.3% Tween®-20. A 100 µl aliquot of a 1:3000 dilution of goat anti-human IgG alkaline phosphatase in wash diluent was added to each well and then incubated at room temperature for 30 minutes. Following six washes with PBS containing 0.3% Tween®-20, p-nitrophenylphosphate substrate (100µl of a 1 mg/ml solution in diethanolamine buffer) was added to each well. The color was allowed to develop for 30 minutes and then read at 444nm in an ELISA plate reader.

Although the foregoing invention has been described in some detail by way of illustration and example for purposes of clarity of understanding, it will be obvious that certain changes and modifications may be practiced within the scope of the appended claims.

## Claims

1. Peptides having fewer than 100 amino acids and comprising at least one epitope defined by one of the following amino acid sequences:
MGARASVLSGGELDP; GELORWEKIRLRPGG; LRPGGKKKYKLKHIV;
LERFAVNPGLLETSE; LGQLQPSLQTGSEEL; GSEELRSLTNTVATL;
TVATLYCVHQRIEIK; RIEIKDTKEALDKIE; GPIAPGQMREPRGSD;
PRGSDIAGTTSTLQE; STLQEQIGWMTNNPP; TNNPPIPVGEITKRW;
IYKRWIILGLNKIVR; QGVGGPGHKARVLAE; RVLAEAMSQVTNTAT;
EGHTARNCRAPRKKG; PRKKGCWKCGKEGHQ; KEGHQMKDCTERQAN;
WPSYKGRPGNPLQSR; QEPIDKELYPLTSLR; WRWGWRWGTMLLGML;
TEKLWVTVYYGVPVW; GVPVWKGATTTLFCA; LWDQSLKPCVKLTPL;
KLTPLCVSLKCTDLK; CTDLKNDTNTNSSSG; ISTSIRGKVQKEYAF;
KTFNGTGPCTNVSTV; NVSTVQCTHGIRPVV; IRPVVSTQLLLNGSL;
NNTRKSIRIQRGPGR; GQIRSCCNITGLLLT; SNNESEIFRPGGGDM;
IEPLGVAPTKAKRRV; AKRRVVQREKRAVGI; RAVGIGALFLGFLGA;
GFLGAAGSTMGAASM; GAASMTLTVQARQLL; ARQLLSGIVQQQNNL;
QQNNLLRAIEAQQHL; AQQHLLQLTVWGIKQ; QLLGIWGCSGKCICT;
KLICTTAVPWNASWS; NASWSNKSLEQIWNN; QIWNNMTWMEWDREI;
WDREINNYTSLIHSL; NQQEKNEQELLELDK; LELDKWASLWNWFNI;
NWFNITNWLWYIKLF; YIKLFIMIVGGLVGL; LPIPRGPDRPEGIEE;
DRDRSIRLVNGSLAL; EALKYWWNLLQYWSQ; QYWSQELKNSAVSLL;

AVAEGTDRVIEVVQG; VTNKGRQKVVPLTNT; PLTNTTNQKTELQAI;
ELQAIYLALQDSGLE; VDKLVSAGIRKILFL; LPPVVAKEIVASCDK;
ASCDKCQLKGEAMHG; LEGKVILVAVHVASG; QGVVESMNKELKKII;
LKKIIGQVRDQAEHL; IRDVGKQMAGDDCVA; QMAGDDCVASRQDED;
MEPVDPRLEPWKHPG; MQPIQIAIVALVVAI; LVVAIIIAIVVWSIV;
KILRQRKIDRLIDRL; LIDRLIERAEDSGNE; MEQAPEDQGPQREPH;
QREPHNEWTLELLEE; GLGQHIYETYGDTWA; FLYQSNPPPNPEGTR;
QIHSISERILGTYLG; LTLDCNEDCGTSGTQ; GLHTGERDWHLGQGV;
LGQGVSIEWRKKRYS; KKRYSTQVDPELADQ; ELADQLIHLYYFDCF;
KVGSLQYLALAALIT; AALITPKKIKPPLPS; and PPLPSVTKLTEDRWN.

2. Peptides as in claim 1, which are substantially free from natural folding.

3. Synthetic peptides as in claim 1, which are substantially free from glycosylation.

4. A synthetic peptide including a sequence substantially the same as one selected from the group consisting of:

MGARASVLSGGELDP; GELORWEKIRLRPGG;
LRPGGKKKYKLKHIV; LERFAVNPGLLETSE; LGQLQPSLQTGSEEL;
GSEELRSLTNTVATL; TVATLYCVHQRIEIK; RIEIKDTKEALDKIE;
GPIAPGQMREPRGSD; PRGSDIAGTTSTLQE; STLQEQIGWMTNNPP;
TNNPPIPVGEITKRW; IYKRWIILGLNKIVR; QGVGGPGHKARVLAE;
RVLAEAMSQVTNTAT; EGHTARNCRAPRKKG; PRKKGCWKCGKEGHQ;
KEGHQMKDCTERQAN; WPSYKGRPGNPLQSR; QEPIDKELYPLTSLR;
WRWGWRWGTMLLGML; TEKLWVTVYYGVPVW; GVPVWKGATTTLFCA;
LWDQSLKPCVKLTPL; KLTPLCVSLKCTDLK; CTDLKNDTNTNSSSG;
ISTSIRGKVQKEYAF; KTFNGTGPCTNVSTV; NVSTVQCTHGIRPVV;
IRPVVSTQLLLNGSL; NNTRKSIRIQRGPGR; GQIRSCCNITGLLLT;
SNNESEIFRPGGGDM; IEPLGVAPTKAKRRV; AKRRVVQREKRAVGI;
RAVGIGALFLGFLGA; GFLGAAGSTMGAASM; GAASMTLTVQARQLL;
ARQLLSGIVQQQNNL; QQNNLLRAIEAQQHL; AQQHLLQLTVWGIKQ;
QLLGIWGCSGKCICT; KLICTTAVPWNASWS; NASWSNKSLEQIWNN;
QIWNNMTWMEWDREI; WDREINNYTSLIHSL; NQQEKNEQELLELDK;
LELDKWASLWNWFNI; NWFNITNWLWYIKLF; YIKLFIMIVGGLVGL;
LPIPRGPDRPEGIEE; DRDRSIRLVNGSLAL; EALKYWWNLLQYWSQ;
QYWSQELKNSAVSLL; AVAEGTDRVIEVVQG; VTNKGRQKVVPLTNT;
PLTNTTNQKTELQAI; ELQAIYLALQDSGLE; VDKLVSAGIRKILFL;
LPPVVAKEIVASCDK; ASCDKCQLKGEAMHG; LEGKVILVAVHVASG;
QGVVESMNKELKKII; LKKIIGQVRDQAEHL; IRDVGKQMAGDDCVA;
QMAGDDCVASRQDED; MEPVDPRLEPWKHPG; MQPIQIAIVALVVAI;
LVVAIIIAIVVWSIV; KILRQRKIDRLIDRL; LIDRLIERAEDSGNE;
MEQAPEDQGPQREPH; QREPHNEWTLELLEE; GLGQHIYETYGDTWA;
FLYQSNPPPNPEGTR; QIHSISERILGTYLG; LTLDCNEDCGTSGTQ;
GLHTGERDWHLGQGV; LGQGVSIEWRKKRYS; KKRYSTQVDPELADQ;
ELADQLIHLYYFDCF; KVGSLQYLALAALIT; AALITPKKIKPPLPS; and
PPLPSVTKLTEDRWN.

5. Synthetic peptides as in claim 1, which are substantially free from natural folding.

6. A synthetic peptide as in claim 4, which is substantially free from glycosylation.

7. Antibody reactive with at least one epitope defined by the following amino acid sequences:

MGARASVLSGGELDP; GELORWEKIRLRPGG; LRPGGKKKYKLKHIV;
LERFAVNPGLLETSE; LGQLQPSLQTGSEEL; GSEELRSLTNTVATL;
TVATLYCVHQRIEIK; RIEIKDTKEALDKIE; GPIAPGQMREPRGSD;
PRGSDIAGTTSTLQE; STLQEQIGWMTNNPP; TNNPPIPVGEITKRW;
IYKRWIILGLNKIVR; QGVGGPGHKARVLAE; RVLAEAMSQVTNTAT;
EGHTARNCRAPRKKG; PRKKGCWKCGKEGHQ; KEGHQMKDCTERQAN;
WPSYKGRPGNPLQSR; QEPIDKELYPLTSLR; WRWGWRWGTMLLGML;
TEKLWVTVYYGVPVW; GVPVWKGATTTLFCA; LWDQSLKPCVKLTPL;
KLTPLCVSLKCTDLK; CTDLKNDTNTNSSSG; ISTSIRGKVQKEYAF;
KTFNGTGPCTNVSTV; NVSTVQCTHGIRPVV; IRPVVSTQLLLNGSL;
NNTRKSIRIQRGPGR; GQIRSCCNITGLLLT; SNNESEIFRPGGGDM;
IEPLGVAPTKAKRRV; AKRRVVQREKRAVGI; RAVGIGALFLGFLGA;

GFLGAAGSTMGAASM; GAASMTLTVQARQLL; ARQLLSGIVQQQNNL;
QQNNLLRAIEAQQHL; AQQHLLQLTVWGIKQ; QLLGIWGCSGKCICT;
KLICTTAVPWNASWS; NASWSNKSLEQIWNN; QIWNNMTWMEWDREI;
WDREINNYTSLIHSL; NQQEKNEQELLELDK; LELDKWASLWNWFNI;
NWFNITNWLWYIKLF; YIKLFIMIVGGLVGL; LPIPRGPDRPEGIEE;
DRDRSIRLVNGSLAL; EALKYWWNLLQYWSQ; QYWSQELKNSAVSLL;
AVAEGTDRVIEVVQG; VTNKGRQKVVPLTNT; PLTNTTNQKTELQAI;
ELQAIYLALQDSGLE; VDKLVSAGIRKILFL; LPPVVAKEIVASCDK;
ASCDKCQLKGEAMHG; LEGKVILVAVHVASG; QGVVESMNKELKKII;
LKKIIGQVRDQAEHL; IRDVGKQMAGDDCVA; QMAGDDCVASRQDED;
MEPVDPRLEPWKHPG; MQPIQIAIVALVVAI; LVVAIIIAIVVWSIV;
KILRQRKIDRLIDRL; LIDRLIERAEDSGNE; MEQAPEDQGPQREPH;
QREPHNEWTLELLEE; GLGQHIYETYGDTWA; FLYQSNPPPNPEGTR;
QIHSISERILGTYLG; LTLDCNEDCGTSGTQ; GLHTGERDWHLGQGV;
LGQGVSIEWRKKRYS; KKRYSTQVDPELADQ; ELADQLIHLYYFDCF;
KVGSLQYLALAALIT; AALITPKKIKPPLPS; and PPLPSVTKLTEDRWN.

8. Monoclonal antibody as in claim 7.

9. Polyclonal antibody as in claim 7.

10. A composition used for eliciting an immune response against HIV-1 infection, said composition comprising a dose of a peptide including a sequence substantially the same as one selected from the group consisting of: MGARASVLSGGELDP;
GELORWEKIRLRPGG; LRPGGKKKYKLKHIV; LERFAVNPGLLETSE;
LGQLQPSLQTGSEEL; GSEELRSLTNTVATL; TVATLYCVHQRIEIK;
RIEIKDTKEALDKIE; GPIAPGQMREPRGSD; PRGSDIAGTTSTLQE;
STLQEQIGWMTNNPP; TNNPPIPVGEITKRW; IYKRWIILGLNKIVR;
QGVGGPGHKARVLAE; RVLAEAMSQVTNTAT; EGHTARNCRAPRKKG;
PRKKGCWKCGKEGHQ; KEGHQMKDCTERQAN; WPSYKGRPGNPLQSR;
QEPIDKELYPLTSLR; WRWGWRWGTMLLGML; TEKLWVTVYYGVPVW;
GVPVWKGATTTLFCA; LWDQSLKPCVKLTPL; KLTPLCVSLKCTDLK;
CTDLKNDTNTNSSSG; ISTSIRGKVQKEYAF; KTFNGTGPCTNVSTV;
NVSTVQCTHGIRPVV; IRPVVSTQLLLNGSL; NNTRKSIRIQRGPGR;
GQIRSCCNITGLLLT; SNNESEIFRPGGGDM; IEPLGVAPTKAKRRV;
AKRRVVQREKRAVGI; RAVGIGALFLGFLGA; GFLGAAGSTMGAASM;
GAASMTLTVQARQLL; ARQLLSGIVQQQNNL; QQNNLLRAIEAQQHL;
AQQHLLQLTVWGIKQ; QLLGIWGCSGKCICT; KLICTTAVPWNASWS;
NASWSNKSLEQIWNN; QIWNNMTWMEWDREI; WDREINNYTSLIHSL;
NQQEKNEQELLELDK; LELDKWASLWNWFNI; NWFNITNWLWYIKLF;
YIKLFIMIVGGLVGL; LPIPRGPDRPEGIEE; DRDRSIRLVNGSLAL;
EALKYWWNLLQYWSQ; QYWSQELKNSAVSLL; AVAEGTDRVIEVVQG;
VTNKGRQKVVPLTNT; PLTNTTNQKTELQAI; ELQAIYLALQDSGLE;
VDKLVSAGIRKILFL; LPPVVAKEIVASCDK; ASCDKCQLKGEAMHG;
LEGKVILVAVHVASG; QGVVESMNKELKKII; LKKIIGQVRDQAEHL;
IRDVGKQMAGDDCVA; QMAGDDCVASRQDED; MEPVDPRLEPWKHPG;
MQPIQIAIVALVVAI; LVVAIIIAIVVWSIV; KILRQRKIDRLIDRL;
LIDRLIERAEDSGNE; MEQAPEDQGPQREPH; QREPHNEWTLELLEE;
GLGQHIYETYGDTWA; FLYQSNPPPNPEGTR; QIHSISERILGTYLG;
LTLDCNEDCGTSGTQ; GLHTGERDWHLGQGV; LGQGVSIEWRKKRYS;
KKRYSTQVDPELADQ; ELADQLIHLYYFDCF; KVGSLQYLALAALIT;
AALITPKKIKPPLPS; and PPLPSVTKLTEDRWN in a physiologically-acceptable carrier, said dose being sufficiently large to elicit a serum neutralizing response in an inoculated host.

11. A composition as in claim 10, further comprising a physiologically-acceptable adjuvant.

12. A solid phase immunoassay capable of detecting anti-pathogen antibodies in a patient sample, said immunoassay comprising:
exposing the patient sample to a solid phase having at least two discrete synthetic peptides immobilized thereon, said peptides encoding immunologically distinct epitopes characteristic of the pathogen; and observing binding between the immobilized peptides and the antibodies in the patient sample.

13. An immunoassay as in claim 12, wherein the pathogen is HIV-1.

14. A solid phase immunoassay as in claim 13, wherein at least one of said immobilized peptides encodes an epitope characteristic of an HIV-1 regulatory protein.

15. A solid phase immunoassay as in claim 12, having at least four discrete synthetic peptides immobilized thereon.

16. A solid phase immunoassay as in claim 12, having at least eight discrete synthetic peptides immobilized thereon.

17. A solid phase immunoassay as in claim 12, wherein the peptides are chemically synthesized linear fragments.

18. A solid phase immunoassay as in claim 12, wherein the peptides are recombinantly produced fragments.

19. A solid phase immunoassay as in claim 12, wherein the synthetic peptides have a length in the range from about 6 to 50 amino acids.


Claims for the following Contracting State : ES

1. A method for obtaining a peptide having fewer than 100 amino acids and comprising at least one epitope defined by one of the following amino acid sequences:
MGARASVLSGGELDP; GELORWEKIRLRPGG; LRPGGKKKYKLKHIV;
LERFAVNPGLLETSE; LGQLQPSLQTGSEEL; GSEELRSLTNTVATL;
TVATLYCVHQRIEIK; RIEIKDTKEALDKIE; GPIAPGQMREPRGSD;
PRGSDIAGTTSTLQE; STLQEQIGWMTNNPP; TNNPPIPVGEITKRW;
IYKRWIILGLNKIVR; QGVGGPGHKARVLAE; RVLAEAMSQVTNTAT;
EGHTARNCRAPRKKG; PRKKGCWKCGKEGHQ; KEGHQMKDCTERQAN;
WPSYKGRPGNPLQSR; QEPIDKELYPLTSLR; WRWGWRWGTMLLGML;
TEKLWVTVYYGVPVW; GVPVWKGATTTLFCA; LWDQSLKPCVKLTPL;
KLTPLCVSLKCTDLK; CTDLKNDTNTNSSSG; ISTSIRGKVQKEYAF;
KTFNGTGPCTNVSTV; NVSTVQCTHGIRPVV; IRPVVSTQLLLNGSL;
NNTRKSIRIQRGPGR; GQIRSCCNITGLLLT; SNNESEIFRPGGGDM;
IEPLGVAPTKAKRRV; AKRRVVQREKRAVGI; RAVGIGALFLGFLGA;
GFLGAAGSTMGAASM; GAASMTLTVQARQLL; ARQLLSGIVQQQNNL;
QQNNLLRAIEAQQHL; AQQHLLQLTVWGIKQ; QLLGIWGCSGKCICT;
KLICTTAVPWNASWS; NASWSNKSLEQIWNN; QIWNNMTWMEWDREI;
WDREINNYTSLIHSL; NQQEKNEQELLELDK; LELDKWASLWNWFNI;
NWFNITNWLWYIKLF; YIKLFIMIVGGLVGL; LPIPRGPDRPEGIEE;
DRDRSIRLVNGSLAL; EALKYWWNLLQYWSQ; QYWSQELKNSAVSLL;
AVAEGTDRVIEVVQG; VTNKGRQKVVPLTNT; PLTNTTNQKTELQAI;
ELQAIYLALQDSGLE; VDKLVSAGIRKILFL; LPPVVAKEIVASCDK;
ASCDKCQLKGEAMHG; LEGKVILVAVHVASG; QGVVESMNKELKKII;
LKKIIGQVRDQAEHL; IRDVGKQMAGDDCVA; QMAGDDCVASRQDED;
MEPVDPRLEPWKHPG; MQPIQIAIVALVVAI; LVVAIIIAIVVWSIV;
KILRQRKIDRLIDRL; LIDRLIERAEDSGNE; MEQAPEDQGPQREPH;
QREPHNEWTLELLEE; GLGQHIYETYGDTWA; FLYQSNPPPNPEGTR;
QIHSISERILGTYLG; LTLDCNEDCGTSGTQ; GLHTGERDWHLGQGV;
LGQGVSIEWRKKRYS; KKRYSTQVDPELADQ; ELADQLIHLYYFDCF;
KVGSLQYLALAALIT; AALITPKKIKPPLPS; and PPLPSVTKLTEDRWN.

2. A method according to claim 1, wherein the peptide is substantially free from natural folding.

3. A method according to claim 1 or claim 2 wherein the peptide is substantially free from glycosylation.

4. A method of synthesizing a peptide which includes a sequence substantially the same as one selected from:
MGARASVLSGGELDP; GELORWEKIRLRPGG;
LRPGGKKKYKLKHIV; LERFAVNPGLLETSE; LGQLQPSLQTGSEEL;
GSEELRSLTNTVATL; TVATLYCVHQRIEIK; RIEIKDTKEALDKIE;
GPIAPGQMREPRGSD; PRGSDIAGTTSTLQE; STLQEQIGWMTNNPP;
TNNPPIPVGEITKRW; IYKRWIILGLNKIVR; QGVGGPGHKARVLAE;
RVLAEAMSQVTNTAT; EGHTARNCRAPRKKG; PRKKGCWKCGKEGHQ;
KEGHQMKDCTERQAN; WPSYKGRPGNPLQSR; QEPIDKELYPLTSLR;
WRWGWRWGTMLLGML; TEKLWVTVYYGVPVW; GVPVWKGATTTLFCA;

LWDQSLKPCVKLTPL; KLTPLCVSLKCTDLK; CTDLKNDTNTNSSSG;
ISTSIRGKVQKEYAF; KTFNGTGPCTNVSTV; NVSTVQCTHGIRPVV;
IRPVVSTQLLLNGSL; NNTRKSIRIQRGPGR; GQIRSCCNITGLLLT;
SNNESEIFRPGGGDM; IEPLGVAPTKAKRRV; AKRRVVQREKRAVGI;
RAVGIGALFLGFLGA; GFLGAAGSTMGAASM; GAASMTLTVQARQLL;
ARQLLSGIVQQQNNL; QQNNLLRAIEAQQHL; AQQHLLQLTVWGIKQ;
QLLGIWGCSGKCICT; KLICTTAVPWNASWS; NASWSNKSLEQIWNN;
QIWNNMTWMEWDREI; WDREINNYTSLIHSL; NQQEKNEQELLELDK;
LELDKWASLWNWFNI; NWFNITNWLWYIKLF; YIKLFIMIVGGLVGL;
LPIPRGPDRPEGIEE; DRDRSIRLVNGSLAL; EALKYWWNLLQYWSQ;
QYWSQELKNSAVSLL; AVAEGTDRVIEVVQG; VTNKGRQKVVPLTNT;
PLTNTTNQKTELQAI; ELQAIYLALQDSGLE; VDKLVSAGIRKILFL;
LPPVVAKEIVASCDK; ASCDKCQLKGEAMHG; LEGKVILVAVHVASG;
QGVVESMNKELKKII; LKKIIGQVRDQAEHL; IRDVGKQMAGDDCVA;
QMAGDDCVASRQDED; MEPVDPRLEPWKHPG; MQPIQIAIVALVVAI;
LVVAIIIAIVVWSIV; KILRQRKIDRLIDRL; LIDRLIERAEDSGNE;
MEQAPEDQGPQREPH; QREPHNEWTLELLEE; GLGQHIYETYGDTWA;
FLYQSNPPPNPEGTR; QIHSISERILGTYLG; LTLDCNEDCGTSGTQ;
GLHTGERDWHLGQGV; LGQGVSIEWRKKRYS; KKRYSTQVDPELADQ;
ELADQLIHLYYFDCF; KVGSLQYLALAALIT; AALITPKKIKPPLPS; and
PPLPSVTKLTEDRWN.

5. A method for synthesizing a peptide as defined in claim 1 or claim 4, wherein the peptide is substantially free from natural folding.

6. A method of synthesizing a peptide according to claim 4, wherein the peptide is substantially free from glycosylation.

7. A method for preparing an antibody reactive with at least one epitope defined by the following amino acid sequences:
MGARASVLSGGELDP; GELORWEKIRLRPGG; LRPGGKKKYKLKHIV;
LERFAVNPGLLETSE; LGQLQPSLQTGSEEL; GSEELRSLTNTVATL;
TVATLYCVHQRIEIK; RIEIKDTKEALDKIE; GPIAPGQMREPRGSD;
PRGSDIAGTTSTLQE; STLQEQIGWMTNNPP; TNNPPIPVGEITKRW;
IYKRWIILGLNKIVR; QGVGGPGHKARVLAE; RVLAEAMSQVTNTAT;
EGHTARNCRAPRKKG; PRKKGCWKCGKEGHQ; KEGHQMKDCTERQAN;
WPSYKGRPGNPLQSR; QEPIDKELYPLTSLR; WRWGWRWGTMLLGML;
TEKLWVTVYYGVPVW; GVPVWKGATTTLFCA; LWDQSLKPCVKLTPL;
KLTPLCVSLKCTDLK; CTDLKNDTNTNSSSG; ISTSIRGKVQKEYAF;
KTFNGTGPCTNVSTV; NVSTVQCTHGIRPVV; IRPVVSTQLLLNGSL;
NNTRKSIRIQRGPGR; GQIRSCCNITGLLLT; SNNESEIFRPGGGDM;
IEPLGVAPTKAKRRV; AKRRVVQREKRAVGI; RAVGIGALFLGFLGA;
GFLGAAGSTMGAASM; GAASMTLTVQARQLL; ARQLLSGIVQQQNNL;
QQNNLLRAIEAQQHL; AQQHLLQLTVWGIKQ; QLLGIWGCSGKCICT;
KLICTTAVPWNASWS; NASWSNKSLEQIWNN; QIWNNMTWMEWDREI;
WDREINNYTSLIHSL; NQQEKNEQELLELDK; LELDKWASLWNWFNI;
NWFNITNWLWYIKLF; YIKLFIMIVGGLVGL; LPIPRGPDRPEGIEE;
DRDRSIRLVNGSLAL; EALKYWWNLLQYWSQ; QYWSQELKNSAVSLL;
AVAEGTDRVIEVVQG; VTNKGRQKVVPLTNT; PLTNTTNQKTELQAI;
ELQAIYLALQDSGLE; VDKLVSAGIRKILFL; LPPVVAKEIVASCDK;
ASCDKCQLKGEAMHG; LEGKVILVAVHVASG; QGVVESMNKELKKII;
LKKIIGQVRDQAEHL; IRDVGKQMAGDDCVA; QMAGDDCVASRQDED;
MEPVDPRLEPWKHPG; MQPIQIAIVALVVAI; LVVAIIIAIVVWSIV;
KILRQRKIDRLIDRL; LIDRLIERAEDSGNE; MEQAPEDQGPQREPH;
QREPHNEWTLELLEE; GLGQHIYETYGDTWA; FLYQSNPPPNPEGTR;
QIHSISERILGTYLG; LTLDCNEDCGTSGTQ; GLHTGERDWHLGQGV;
LGQGVSIEWRKKRYS; KKRYSTQVDPELADQ; ELADQLIHLYYFDCF;
KVGSLQYLALAALIT; AALITPKKIKPPLPS; and PPLPSVTKLTEDRWN.

8. A method for preparing a monoclonal antibody reactive with at least one epitope as defined in claim 7.

9. A method for preparing a polyclonal antibody reactive with at least one epitope as defined in claim 7.

10. A process for preparing a composition for eliciting an immune response against HIV-1 infection, said process comprising combining a dose of a peptide including a sequence substantially the same as one selected from:

MGARASVLSGGELDP;
GELORWEKIRLRPGG; LRPGGKKKYKLKHIV; LERFAVNPGLLETSE;
LGQLQPSLQTGSEEL; GSEELRSLTNTVATL; TVATLYCVHQRIEIK;
RIEIKDTKEALDKIE; GPIAPGQMREPRGSD; PRGSDIAGTTSTLQE;
STLQEQIGWMTNNPP; TNNPPIPVGEITKRW; IYKRWIILGLNKIVR;
QGVGGPGHKARVLAE; RVLAEAMSQVTNTAT; EGHTARNCRAPRKKG;
PRKKGCWKCGKEGHQ; KEGHQMKDCTERQAN; WPSYKGRPGNPLQSR;
QEPIDKELYPLTSLR; WRWGWRWGTMLLGML; TEKLWVTVYYGVPVW;
GVPVWKGATTTLFCA; LWDQSLKPCVKLTPL; KLTPLCVSLKCTDLK;
CTDLKNDTNTNSSSG; ISTSIRGKVQKEYAF; KTFNGTGPCTNVSTV;
NVSTVQCTHGIRPVV; IRPVVSTQLLLNGSL; NNTRKSIRIQRGPGR;
GQIRSCCNITGLLLT; SNNESEIFRPGGGDM; IEPLGVAPTKAKRRV;
AKRRVVQREKRAVGI; RAVGIGALFLGFLGA; GFLGAAGSTMGAASM;
GAASMTLTVQARQLL; ARQLLSGIVQQQNNL; QQNNLLRAIEAQQHL;
AQQHLLQLTVWGIKQ; QLLGIWGCSGKCICT; KLICTTAVPWNASWS;
NASWSNKSLEQIWNN; QIWNNMTWMEWDREI; WDREINNYTSLIHSL;
NQQEKNEQELLELDK; LELDKWASLWNWFNI; NWFNITNWLWYIKLF;
YIKLFIMIVGGLVGL; LPIPRGPDRPEGIEE; DRDRSIRLVNGSLAL;
EALKYWWNLLQYWSQ; QYWSQELKNSAVSLL; AVAEGTDRVIEVVQG;
VTNKGRQKVVPLTNT; PLTNTTNQKTELQAI; ELQAIYLALQDSGLE;
VDKLVSAGIRKILFL; LPPVVAKEIVASCDK; ASCDKCQLKGEAMHG;
LEGKVILVAVHVASG; QGVVESMNKELKKII; LKKIIGQVRDQAEHL;
IRDVGKQMAGDDCVA; QMAGDDCVASRQDED; MEPVDPRLEPWKHPG;
MQPIQIAIVALVVAI; LVVAIIIAIVVWSIV; KILRQRKIDRLIDRL;
LIDRLIERAEDSGNE; MEQAPEDQGPQREPH; QREPHNEWTLELLEE;
GLGQHIYETYGDTWA; FLYQSNPPPNPEGTR; QIHSISERILGTYLG;
LTLDCNEDCGTSGTQ; GLHTGERDWHLGQGV; LGQGVSIEWRKKRYS;
KKRYSTQVDPELADQ; ELADQLIHLYYFDCF; KVGSLQYLALAALIT;
AALITPKKIKPPLPS; and PPLPSVTKLTEDRWN and a physiologically-acceptable carrier, said does being sufficiently large to elicit a serum neutralizing response in an inoculated host.

11. A process according to claim 10, wherein a physiologically-acceptable adjuvant is also combined with the dose and the carrier.

12. A solid phase immunoassay capable of detecting anti-pathogen antibodies in a patient sample, said immunoassay comprising:
exposing the patient sample to a solid phase having at least two discrete synthetic peptides immobilized thereon, said peptides encoding immunologically distinct epitopes characteristic of the pathogen; and observing binding between the immobilized peptides and the antibodies in the patient sample.

13. An immunoassay as in claim 12, wherein the pathogen is HIV-1.

14. A solid phase immunoassay as in claim 13, wherein at least one of said immobilized peptides encodes an epitope characteristic of an HIV-1 regulatory protein.

15. A solid phase immunoassay as in claim 12, having at least four discrete synthetic peptides immobilized thereon.

16. A solid phase immunoassay as in claim 12, having at least eight discrete synthetic peptides immobilized thereon.

17. A solid phase immunoassay as in claim 12, wherein the peptides are chemically synthesized linear fragments.

18. A solid phase immunoassay as in claim 12, wherein the peptides are recombinantly produced fragments.

19. A solid phase immunoassay as in claim 12, wherein the synthetic peptides have a length in the range from about 6 to 50 amino acids.